# EUROPEAN PATENT SPECIFICATION

(11) **EP 1 435 247 B1**
(45) Date of publication and mention of the grant of the patent: **02.11.2006**
(21) Application number: 03250033.2
(22) Date of filing: 06.01.2003
(51) Int. Cl.: A61L 15/22

(54) **Wound dressings comprising an alginate fabric and a superabsorbent**
Alginatgewebe und Superabsorbens enthaltendes Wundpflaster
Pansements comprenant un tissu d'alginate et un superabsorbant

(43) Date of publication of application: 07.07.2004
(73) Proprietor: Speciality Fibres and Materials Limited, Nottingham, Nottinghamshire NG1 6FZ (GB)
(72) Inventor: Ferguson, Paul John, Walsgrave, Coventry CV2 2NG (GB); Bray, Roger, Whitestone, Nuneaton, CV11 6TU (GB)
(74) Representative: Spencer, Michael David

(56) References cited:
- EP-A- 0 599 589
- EP-A- 0 642 779
- WO-A-96/13282
- GB-A- 2 221 620
- GB-A- 2 377 177
- RU-C- 2 031 661
- DATABASE WPI Section Ch, Week 199544 Derwent Publications Ltd., London, GB; Class A96, AN 1995-342809 XP002245199 & RU 2 031 661 C (EKOMEDSERVIS RES PRODN ENTERP), 27 March 1995 (1995-03-27)

## Description

The present invention relates to wound dressings, in particular to dressings suitable for and advantageous for use on highly exuding wounds, especially bleeding wounds or ulcers.

Wound dressings composed of or containing alginate have been known for many years. For example, US-A-2,512,616 and US-A-2,688,586 disclose haemostatic surgical dressings comprising a fibrous alginate material such as calcium alginate or sodium calcium alginate. GB-A-1,394, 742 discloses a surgical dressing material comprising a layer of knitted gauze comprising alginic material adhered to a layer of fibrous backing material such as muslin, such that the dressing material is of lower flexibility and stretchability than the gauze itself. Alternatively, as in US-A-5,470,576 or GB-A-2,221,620, alginate material may be impregnated into or coated onto a fibrous substrate to form a wound dressing material.

Alginates can be divided into two types according to their behaviour in pure water, namely so-called water-soluble alginates and water-insoluble alginates. The present invention is solely concerned with water-insoluble alginates, which, if made firstly into fibres and then into a fabric, could be dipped into pure water and would merely get wet without dissolving or forming a gel. Such fabrics could then subsequently be dried and would be unchanged in form from the initial fabric. In contrast, if a water-soluble alginate were to be made into a fabric and dipped into pure water it would dissolve. However, although a fabric of a water-insoluble alginate would not dissolve in pure water, if the same fabric were to be dipped into a saline solution containing sodium ions, such as a 0.9% NaCl solution, the alginate would form a gel. It can be seen therefore that there are fundamental differences between these two forms of alginate. Further details on the differences are given below. As used hereinafter, the term "water-insoluble" in relation to alginates refers to insolubility in pure water and the term "gel-forming alginate" is used to mean a water-insoluble alginate which forms a gel in 0.9% NaCl saline solution.

In use, wound dressings comprising alginate have haemostatic properties and accordingly help to staunch bleeding. Gel-forming alginate wound dressings have low adherence to wounds, and they can be easily removed from wounds without causing trauma and without leaving potentially harmful dressing fragments in the wounds.

It is well-known that gel-forming substances such as alginate are prone to gel-block, especially when suddenly exposed to large quantities of liquid such as blood. In gel-blocking, the part of the gel-forming substance first exposed to a liquid swells so rapidly and extensively that it blocks transmission of the liquid to and absorption of the liquid by the parts of the gel-forming substance not yet reached by the liquid. Such behaviour may be advantageous in wound dressings to the extent that it reduces the lateral spread of absorbed exudate, helping to prevent the maceration of healthy skin surrounding the wound. However, if gel-blocking is too severe, then it can serve to prevent the full use of the gel-forming substance in the dressing and can in this respect be disadvantageous. Alginate dressings exhibit a moderate degree of gel-blocking that is appropriate for their use in contact with wounds.

However, although gel-forming alginate dressings exhibit a good absorption capacity for liquids such as wound exudates, they have a relatively low liquid-holding or liquid-retention capacity. Therefore, liquid absorbed into a gel-forming alginate dressing is easily expressed by squeezing.

Consequently, although gel-forming alginate dressings can be used successfully on highly exuding wounds, we have discovered that these dressings can be improved upon, in particular for use on bleeding wounds, where the transmission of agents carrying diseases such as hepatitis and HIV syndrome is of concern. Ideally, the exudate or blood should be absorbed and then locked away in the dressing structure with minimal possibility for the re-expression of liquid.

Superabsorbent materials such as fibres are also known. For example US-A-4,392,908 and US-A-5,582,786 disclose such materials. Superabsorbent materials have high liquid-holding capacity, being capable of absorbing and retaining even under load several multiples of their own weight of aqueous liquids. It is difficult to express the bulk of such absorbed liquor. The term superabsorbent has been used in the past to describe materials having a variety of properties, so care is need to ensure that the term is properly qualified when using it in any specific context. Superabsorbent materials are known which have free-swell absorption figures of 25 g/ g or more and retention figures for free-swollen materials of 15 g/ g or more under loads of 35 gcm⁻², and only such materials are superabsorbents for the purpose of the present invention. Such highly absorbent materials are used in absorbent personal products, for example tampons, disposable diapers, sanitary napkins and incontinence pads, because of their ability to "suck up" large quantities of bodily fluids and lock them away whilst keeping the personal products relatively dry.

However, the above-mentioned advantage of such a superabsorbent material becomes a significant disadvantage in relation to wounds. First of all, the tendency of such a superabsorbent material to suck up liquid such as blood would tend to encourage a wound contacted by the superabsorbent material to bleed. (This is akin to the well-known phenomenon that a small open cut stops bleeding quickly if left alone whereas blood flow is encouraged if the blood is continually dabbed away.) Secondly, when a wound does eventually stop bleeding, the superabsorbent material continuing to be in contact with the wound would tend to dry out the wound because of its ability and tendency to absorb and retain liquid from the drying wound. This forced drying out causes pain by drying out the nerve endings exposed in the wound. It also reduces the healing which the wound would naturally carry out in a moist environment. Such superabsorbent materials have a high adherence to wounds and are consequently difficult to remove from contact with a wound.

In addition, such superabsorbent materials in fibre form tend, especially when wet, to be very brittle materials but materials which still retain their fibrous form. Therefore, there would be concern that fragments may be left at the wound site if the material is placed directly against the wound.

Consequently, such highly absorbing superabsorbent materials have been contra-indicated for use in wound dressings where they would come into direct contact with the wound. Such superabsorbent materials in their pure state are also commonly prone to rapid and severe gel-blocking.

US-A-5,674,524 discloses an alginate dressing of improved absorbency comprising a fibrous alginate layer needle-punched to a non-alginate backing web, but it does not mention any superabsorbent material present in the backing web.

US-A-5,998,692 discloses a wound dressing for use on high exuding wounds comprising a breathable film, an absorbent layer having high fluid holding capacity located on the film side remote from the wound, and optionally a further absorbent layer located on the film side proximal to the wound, but it does not mention any superabsorbent material present in the absorbent layers.

EP-A-0,642,779 discloses a wound dressing comprising a multi-layer laminate having a wound contact layer with a high wet-strength, optionally including a wound-healing promoter such as a water-soluble sodium calcium alginate, a core layer containing superabsorbent material, and a top surface layer of a material capable of stretching at least uniaxially. The superabsorbent material is generally a water-insoluble but water-swellable polymeric substance capable of absorbing water in an amount which is at least 10 times the weight of the substance in its dry form, but no further figures are given. Modified polysaccharides are the only superabsorbent materials mentioned.

EP-A-0,599,589 discloses wound dressings comprising a molecular filtration membrane separating a wound contact layer of wound-friendly bioabsorbable material from an absorbent layer which can comprise a wide range of absorbent materials such as fabrics, superabsorbents, foams or particulate absorbents and normally has an absorbency in the range of from 500 gm⁻² to 10,000 gm⁻² but there is no indication of the absorbency of the material itself, nor does it specify dressing thickness.

We have found that good results can be achieved for the treatment of wounds which are bleeding or otherwise highly exuding by the use of a wound dressing in which the wound-contacting surface is an alginate fabric in which the alginate is a water-insoluble material but one which is gel-forming in a saline solution (0.9% NaCl) and in which a backing layer including a fibrous superabsorbent material and having good absorbency and liquid retention is provided in direct physical contact with the gel-forming fabric.

According to the invention there is provided a wound dressing which comprises a wound-contacting layer of an alginate fabric which is insoluble in water and which is gel-forming in saline solution (0.9% NaCl), backed by and in liquid contact with a layer including a superabsorbent material, characterized in that the superabsorbent material is fibrous and is in direct physical contact with the layer of alginate fabric, in that the superabsorbent material has a free-swell absorption of at least 25 g of saline (0.9% NaCl) solution per g of superabsorbent material and a retention of absorbed saline (0.9% NaCl) solution under a load of 35 gcm⁻² of at least 15 g per g of superabsorbent material, and in that the layer including the superabsorbent material has a free-swell absorption of at least 10 g of saline (0.9% NaCl) solution per g of the layer and a retention of absorbed saline (0.9% NaCl) solution under a load of 35 gcm⁻² of at least 5 g per g of the layer.

Gel-forming alginate fabrics are haemostatic and low-adherent. In use, the gel-forming alginate face of the dressing is placed against and in contact with the wound. The wound dressing therefore has low adherence to the wound, and it can be easily removed therefrom without causing trauma and without leaving dressing fragments in the wound.

The gel-forming alginate fabric may be a woven or knitted fabric, but it is preferably a nonwoven, e.g. needlepunched, fabric. The basis weight of the gel-forming alginate fabric is preferably in the range from 25 - 200 gm⁻², more preferably 50 to 150 gm⁻², most preferably 75 to 150 gm⁻². Typical gel-forming alginate fibres have a free-swell absorption of the order of about 20 g/ g, that is to say they absorb about 20 g of saline solution (0.9% NaCl) per g of fibre in the unrestrained state. The gel-forming alginate fibre in the alginate fabric is typically calcium alginate or calcium sodium alginate. Dressings comprising gel-forming alginate as wound-contacting layer can, if designed appropriately, exhibit a useful degree of gel-blocking such that the lateral spread of absorbed exudate or blood in the wound-contacting layer is reduced and yet transmission of such liquids through the wound-contacting layer to the layer including a superabsorbent material remains readily possible. This desirable balance of properties can be achieved by selecting a suitable calcium:sodium ratio and suitable ratio of mannuronate to guluronate monomer residues (M:G ratio) for the alginate polymer. Preferred gel-forming alginate fabrics for the dressings of the invention are those made with so-called high M alginate fibres, which contain an M:G ratio above 50:50, for example 60:40, together with a calcium:sodium ratio which is high, above 90:10 or 10:1, for example 50:1. The gel-forming alginate fibre may contain minor proportions of therapeutically-active metal ions such as zinc or silver. The alginate fibre may be medicated. The titre of the gel-forming alginate fibre is preferably in the range from 1.5 to 5 decitex. Such fibres are known and are commercially available.

The superabsorbent material in the dressing is in fibre form, and the layer including the superabsorbent material is preferably a fabric. Such a fabric may be a woven or nonwoven, e.g. needlepunched or air-laid, fabric. The basis weight of such a fabric is preferably in the range from 50 to 350 gm⁻², more preferably 150 to 250 gm⁻². The invention thus provides a dressing which can usefully be used at thicknesses of as low as 2-4 mm. In some cases thick dressings are preferred, for example of 5 to 6 mm thickness or more, so as to protect the wound, particularly ulcerating wounds. In other cases thinner dressings, for example of less than 4.5 mm thickness, are desirable as they interfere less with the day-to-day activities of the patient, such as putting on clothes. Although producing thick dressings which are more absorbent just involves the use of greater amounts of absorbing material, this option is not open to manufacturers of thinner dressings, without loss of thinness.

The superabsorbent fibrous material, is one which has a free-swell absorption of at least 25 g/g, that is to say it absorbs at least 25 g of saline solution (0.9% NaCl) per g of superabsorbent material in the unrestrained state, and which has a retention under a load of 35 gcm⁻² of at least 15 g/g, that is to say it retains at least 15 g of saline (0.9% NaCl) solution which has been absorbed by the material in unrestrained state per g of superabsorbent material when the material containing the absorbed saline solution is subjected to a load of 35 gcm⁻² (0.5 psi).

The free-swell absorption of the superabsorbent material is desirably at least 30 g/g and may for example be 35 g/ g or more, preferably at least 40 g/ g, such as 45 g/g or more, up to 50 g/g or more. The retention by the superabsorbent material under a load of 35 gcm⁻² of previously absorbed saline solution is desirably at least 20 g/ g, such as 22 g/g or more, preferably at least 30 g/g, but does not exceed the free-swell absorption.

The superabsorbent material is preferably a synthetic polymer such as polyacrylate, for example the polyacrylate fibre disclosed in US-A-5,582,786, the contents of which are hereby incorporated by this reference. Such a material is commercially available from Technical Absorbents Limited under the Registered Trade Mark OASIS.

As the superabsorbent material does not come into physical contact with the wound in the wound dressings of the invention, its high adherence to wounds and consequent difficulty of removal therefrom is not a disadvantage of the dressings of the invention. Moreover, even though superabsorbent materials as such are prone to rapid and severe gel-blocking, this is not a disadvantage where the superabsorbent material is used as part of a backing to a gel-forming alginate fabric of lower liquid retention, which acts as the wound-contacting layer.

The layer including the superabsorbent material has a free-swell absorption of at least 10 g of saline solution per g of the layer, usually 10 to 50 g/g, preferably 15 to 30 g/ g, and a retention of absorbed saline solution of at least 5 g per g of the layer under a load of 35 gcm⁻², usually 5 to 40 g/ g, preferably 5 to 20 g/g.

The layer including the superabsorbent material or the layer of gel-forming alginate fabric may also contain one or more anti-bacterial agents to prevent or treat infection or to restrict odour formation. These agents may be bonded to or incorporated within the superabsorbent material or the gel-forming alginate fabric or any other components of the layer concerned.

The dressings of the invention therefore provide a desirable combination of a layer designed for wound contact backed by a layer designed for absorption and retention of liquid. The gel-forming alginate wound-contacting surface provides a moist environment for optimum healing and avoidance of pain associated with drying out of the wound and a haemostatic effect to reduce and stop further bleeding. The superabsorbent material absorbs and locks away large quantities of blood and yet tends to retain its fibrous nature, whereas the gel formed by the gel-forming alginate material can be removed or washed away from the wound without leaving fibre particles behind in the wound. In the dressings of the invention, liquids such as blood can pass through the gel-forming alginate layer without causing an undesirable degree of gel blocking and be absorbed by the superabsorbent layer.

The gel-forming alginate fabric and the superabsorbent material are in physical contact with one another. It will be appreciated that they must be in liquid contact, i.e. in such contact that liquid can pass between them.

The dressing may be a unitary fabric formed by needlepunching together webs of gel-forming alginate fibre and of a fabric containing the superabsorbent fibre.

The layer including the superabsorbent material may include a low melting point polymer so that it and the wound-contacting layer can be thermally bonded together, for example by passing them between heated rollers.

The dressing may additionally comprise a liquid-impermeable and breathable outer backing layer over the layer including the superabsorbent material on the side remote from the wound-contacting layer. The dressing may additionally comprise means for attachment to a patient, such as an adhesive layer extending beyond the gel-forming alginate fabric.

The wound dressings of the invention absorb blood or other wound exudate with minimal leakage, even if applied under pressure.

The wound dressings are particularly suitable for first aid treatment. They are well suited to use outside a medically-equipped location such as a hospital or surgery. For example, they can be applied to bleeding wounds arising during physical contact sports. They can be applied by unskilled or semiskilled persons. There are nowadays justified fears of contact with the blood of other persons because of the risk of transmission of agents which cause diseases such as hepatitis and HIV syndrome. The dressings of the invention serve both to absorb blood and to staunch blood flow and thus help to minimise such risks.

The following Table gives data on free swell, retention and absorbency under load (AUL) for two superabsorbent fibres which can be used and for one gel-forming alginate fibre which can be used in the dressings of the invention.

| Fibre | Oasis Type 101/102 (a polyacrylate fibre available from Technical Absorbents Ltd) | Oasis Type 121/122 (another polyacrylate fibre available from Technical Absorbents Ltd, more highly cross linked than Type 101/102) | Calcium Alginate (an alginate with calcium sodium ratio 50:1 and M:G ratio 60:40 available from Acordis Speciality Fibres Ltd.) |
|---|---|---|---|
| Free swell absorption | 45 | 27 | 21 |
| Retention | 35 | 18 | 11 |
| AUL | 21 | 17 | 5 |
| All figures in the Table are g of saline (0.9% by weight Na Cl) solution per g of material. Free swell = amount of saline solution absorbed in unrestrained state. Retention = a measurement made by allowing the fibre to absorb the saline solution under free-swell conditions, followed by application of a load at a pressure of 35gcm⁻² (0.5psi) in order to determine the liquid-retaining capacity under a later applied pressure. AUL = absorption of saline solution under an applied load of 22gcm⁻² (0.3 psi). The difference between retention and AUL is, therefore, that retention is measured by first absorbing under no load and then applying a load whereas AUL requires the product to absorb whilst under a load. Thus, in practice for the present invention, it is the difference between a wound dressing being compressed on application and absorbing whilst compressed (AUL) and a wound dressing being applied to a wound more loosely and then being pressurized after it has absorbed liquid, for example by being physically pressed or by a patient wearing the dressing rolling over onto it (Retention). | | | |

The superabsorbent fibre (OASIS), once saturated, can be re-dried to regain its fibrous form. In its saturated form it comprises swollen fibres. Gel-forming alginate is not superabsorbent and absorbs saline to become an amorphous gel sheet with relatively little fibrous character retained. On drying, it does not regain its fibrous form but produces brittle sheets.

The invention will now be further described with reference to the following Examples:

### Example 1

A wound dressing was made up as a composite material comprising a wound-contacting layer and a layer including a superabsorbent material. Layer 1, the wound-contacting layer, consisted of a low-adherent gel-forming fabric made from calcium alginate fibre (calcium:sodium ratio 50:1, M:G ratio 60:40) (Acordis Speciality Fibres Ltd.). The fabric was manufactured at a basis weight of 70 gm⁻² by a carding and crossfolding technique, followed by needlebonding, to confer a degree of integrity to the fabric.

Layer 2 contained a superabsorbent material designed to absorb and retain large quantities of fluid. The layer used was supplied as a fabric by Lantor (UK) Ltd. and designated as Material Reference 48-10-12. The layer had a basis weight of 190 gm⁻² and was composed of an intimate blend of fibres which had been carded and needlebonded together in the ratio of 40% polyacrylate (Oasis superabsorbent Type 101/102) (Technical Absorbents Ltd.) / 50% viscose / 10% polyolefin, by weight.

Layer 1 and layer 2 were bonded together by passing the two fabrics through a needle loom and needling through layer 1 and down into layer 2.

The conditions used were as follows:
Needle punch density - 18 ncm⁻²
Needle penetration -10 mm
Throughput speed - 2.5 m/min

The composite fabric had a thickness of 4.8 mm. It was tested for its absorbency and liquid retention in a sodium chloride and calcium chloride solution formed by dissolving 8.298 g of sodium chloride and 0.368 g of calcium chloride dihydrate in sufficient distilled water to produce 1000 ml of solution. Absorbency (free swell) was measured using the BP "Petri Dish" Test for Absorbency of Alginate Dressings (BP 1993 addendum 1995) except that the amount of liquor used was 80 times the weight of the dressing, instead of the standard 40 times, due to the high absorbency of the samples under test.

Liquid retention was measured as follows: wet samples were taken from the BP "Petri Dish" Absorbency Test and placed on a sheet of absorbent (blue) roll which had been folded in half three times to give a total of 8 layers. A perspex plate and a weight were placed on top to give a total weight of 1358 g (which is equivalent to 54.3 gcm⁻²) for 1 minute. After this time the weight and plate were removed and the sample re-weighed. Liquid retained by the sample was then calculated.

Using this method of measurement, the absorbency of the composite fabric was measured as 14 g/ g (46 g/100 cm²) with a liquid retention of 8.5 g/ g.

Layer 2 alone was tested in the same way. Its absorbency was measured as 20 g/ g (44 g/100 cm²), with a liquid retention of 11.5 g/ g.

### Example 2

A wound dressing was made up as a composite material comprising a wound-contacting layer and a layer including a superabsorbent material. Layer 1, the wound-contacting layer, consisted of the low-adherent gel-forming fabric used as layer 1 in Example 1.

Layer 2 contained a superabsorbent material designed to absorb and retain large quantities of fluid. The layer used was supplied as a fabric by Dan-Webforming International A/S and designated as Material Reference D3/09/02. The material had a basis weight of 180 gm⁻² and was composed of a blend of fibres in the ratio of 30% polyacrylate (Oasis superabsorbent Type 101/102) (Technical Absorbents Ltd.) / 47% cellulose fibres / 23% polyolefin thermoplastic binder fibres, by weight. The fibres had been air-laid and thermally bonded to form the fabric.

Layer 1 and layer 2 were bonded together by passing the two fabrics through a needle loom and needling through layer 1 and down into layer 2.

The conditions used were as follows:
Needle punch density - 23 ncm⁻²
Needle penetration - 9 mm
Throughput speed - 2 m/ min

The composite fabric had a thickness of 2.7 mm. It was tested for its absorbency and liquid retention in the same way as the composite fabric of Example 1.

Using this method of measurement, the absorbency of the composite fabric was measured as 18 g/ g (46 g/100cm²) with a liquid retention of 9 g/g.

Layer 2 alone was tested in the same way. Its absorbency was measured at 20 g/ g (38 g/100cm²) with a liquid retention of 10 g/g.

## Claims

1. A wound dressing which comprises a wound-contacting layer of an alginate fabric which is insoluble in water and which is gel-forming in saline solution (0.9% NaCl), backed by and in liquid contact with a layer including a superabsorbent material, **characterised in that** the superabsorbent material is fibrous and is in direct physical contact with the layer of alginate fabric, **in that** the superabsorbent material has a free-swell absorption of at least 25 g of saline solution (0.9% NaCl) per g of superabsorbent material and a retention of absorbed saline solution (0.9% NaCl) under a load of 35 gcm⁻² of at least 15 g per g of superabsorbent material, and **in that** the layer including the superabsorbent material has a free-swell absorption of at least 10 g of saline solution (0.9% NaCl) per g of the layer and a retention of absorbed saline solution (0.9% NaCl) under a load of 35 gcm⁻² of at least 5 g per g of the layer.

2. A wound dressing as claimed in claim 1, in which the fibrous superabsorbent material has a free-swell absorption of at least 30 g of saline solution (0.9% NaCl) per g of fibre and a retention of absorbed saline solution (0.9% NaCl) of at least 20 g per g of fibre under a load of 35gcm⁻².

3. A wound dressing as claimed in claim 1 or claim 2, in which the layer including the superabsorbent material is a layer of fabric, the fabric being formed at least in part from the fibrous superabsorbent material.

4. A wound dressing as claimed in claim 3, in which the fabric has a basis weight in the range 50-350 g/m², preferably 150-250 g/m²

5. A wound dressing as claimed in any of claims 1 to 4 in which the superabsorbent material has a free-swell absorption of at least 40 g of saline solution (0.9% NaCl) per g of superabsorbent material and a retention of absorbed saline solution (0.9% NaCl) of at least 30 g of superabsorbent material under a load of 35gcm⁻².

6. A wound dressing as claimed in any of claims 1 to 5, in which the layer including the superabsorbent material has a free-swell absorption of 15 to 30 g of saline solution (0.9% NaCl) per g of the layer and a retention of absorbed saline solution (0.9% NaCl) of 5 to 20 g per g of the layer.

7. A wound dressing as claimed in any of claims 1 to 6, having a thickness of 2 to 4 mm.

8. A wound dressing as claimed in any of claims 1 to 7, in which either the alginate fabric or the layer including the superabsorbent material also contains an anti-bacterial agent to prevent or treat infection or to restrict odour formation.

9. A wound dressing as claimed in any of claims 1 to 8, in which the superabsorbent material is polyacrylate.

10. A wound dressing as claimed in any of claims 1 to 9, in which the dressing is a unitary needlepunched fabric of an alginate fibre web and a fabric containing a fibrous superabsorbent material.

## Patentansprüche

1. Wundverband, umfassend eine Wundkontaktschicht aus einem Alginatstoff, der wasserunlöslich ist und der in Salzlösung (0,9% NaCl) ein Gel bildet, und die von einer ein superabsorbierendes Material enthaltenden Schicht gestützt wird, und mit dieser in Flüssigkeitskontakt steht,
**dadurch gekennzeichnet, dass**
das superabsorbierende Material faserförmig ist und sich in direktem physikalischen Kontakt mit der Schicht des Alginatstoffs befindet,
das superabsorbierende Material eine Quellungs-Aufnahmefähigkeit von mindestens 25 g Salzlösung (0,9% NaCl) pro g superabsorbierendes Material und eine Retention von absorbierter Salzlösung (0,9% NaCl) unter einer Last von 35 gcm⁻² von mindestens 15 g pro g superabsorbierendem Material aufweist, und
die Schicht, die das superabsorbierende Material enthält, eine Quellungs-Aufnahmefähigkeit von mindestens 10 g Salzlösung (0,9% NaCl) pro g Schicht und eine Retention von absorbierter Salzlösung (0,9% NaCl) unter einer Last von 35 gcm⁻² von mindestens 5 g pro g Schicht aufweist.

2. Wundverband nach Anspruch 1, wobei das faserförmige superabsorbierende Material eine Quellungs-Aufnahmefähigkeit von mindestens 30 g Salzlösung (0,9% NaCl) pro g Faser und eine Retention von absorbierter Salzlösung (0,9% NaCl) von mindestens 20 g pro g Faser unter einer Last von 35 gcm⁻² aufweist.

3. Wundverband nach Anspruch 1 oder 2, wobei die Schicht, die das superabsorbierende Material enthält, eine Stoffschicht ist, wobei der Stoff zumindest teilweise aus dem faserförmigen superabsorbierenden Material hergestellt ist.

4. Wundverband nach Anspruch 3, wobei der Stoff ein Flächengewicht im Bereich von 50 bis 350 g/m², vorzugsweise 150 bis 250 g/m², aufweist.

5. Wundverband nach einem der Ansprüche 1 bis 4, wobei das superabsorbierende Material eine Quellungs-Aufnahmefähigkeit von mindestens 40 g Salzlösung (0,9% NaCl) pro g superabsorbierendes Material und eine Retention von absorbierter Salzlösung (0,9% NaCl) von mindestens 30 g superabsorbierendem Material unter einer Last von 35 gcm⁻² aufweist.

6. Wundverband nach einem der Ansprüche 1 bis 5, wobei die Schicht, die das superabsorbierende Material enthält, eine Quellungs-Aufnahmefähigkeit von 15 bis 30 g Salzlösung (0,9% NaCl) pro g Schicht und eine Retention von absorbierter Salzlösung (0,9% NaCl) von 5 bis 20 g pro g Schicht aufweist.

7. Wundverband nach einem der Ansprüche 1 bis 6 mit einer Dicke von 2 bis 4 mm.

8. Wundverband nach einem der Ansprüche 1 bis 7, wobei entweder der Alginatstoff oder die Schicht, die das superabsorbierende Material enthält, auch ein antibakterielles Mittel enthält, damit eine Infektion verhindert oder behandelt wird, oder die Geruchsentstehung eingeschränkt wird.

9. Wundverband nach einem der Ansprüche 1 bis 8, wobei das superabsorbierende Material Polyacrylat ist.

10. Wundverband nach einem der Ansprüche 1 bis 9, wobei der Verband ein einheitliches Nadelvlies aus einem Alginatfasergewebe und einem Stoff ist, der ein faserförmiges superabsorbierendes Material enthält.

## Revendications

1. Pansement pour plaie qui comprend une couche de contact avec la plaie d'un tissu d'alginate qui est insoluble dans l'eau et qui forme un gel dans une solution saline (0,9% de NaCl), renforcé par et en contact liquide avec une couche comprenant un matériau superabsorbant, **caractérisé en ce que** le matériau superabsorbant est fibreux et est en contact physique direct avec la couche de tissu d'alginate, **en ce que** le matériau superabsorbant a un pouvoir absorbant en gonflement libre de 25 g de solution saline (0,9% de NaCl) au moins par gramme de matériau superabsorbant et une rétention de solution saline (0,9% de NaCl ) absorbée sous une charge de 35 gcm⁻² de 15 g au moins par gramme de matériau superabsorbant, et **en ce que** la couche comprenant le matériau superabsorbant a un pouvoir absorbant en gonflement libre de 10 g au moins de solution saline (0,9% de NaCl) par gramme de couche et une rétention de solution saline (0,9% de NaCl) absorbée sous une charge de 35 gcm⁻² de 5 g au moins par gramme de couche.

2. Pansement pour plaie selon la revendication 1, dans lequel le matériau superabsorbant fibreux a un pouvoir absorbant en gonflement libre de 30 g au moins de solution saline (0,9% de NaCl) par gramme de fibre et une rétention de la solution saline (0,9% de NaCl) absorbée de 20 g au moins par gramme de fibre sous une charge de 35 gcm⁻².

3. Pansement pour plaie selon la revendication 1 ou la revendication 2, dans lequel la couche comprenant le matériau superabsorbant est une couche de tissu, le tissu étant formé, au moins en partie, du matériau superabsorbant fibreux.

4. Pansement pour plaie selon la revendication 3, dans lequel le tissu a une masse surfacique dans la fourchette de 50 à 350 g/m², de préférence de 150 à 250 g/m².

5. Pansement pour plaie selon l'une quelconque des revendications 1 à 4, dans lequel le matériau superabsorbant a un pouvoir absorbant en gonflement libre de 40 g au moins de solution saline (0,9% de NaCl) par gramme de matériau superabsorbant et une rétention de solution saline (0,9% de NaCl) absorbée de 30 g au moins de matériau superabsorbant sous une charge de 35 gcm⁻².

6. Pansement pour plaie selon l'une quelconque des revendications 1 à 5, dans lequel la couche comprenant le matériau superabsorbant a un pouvoir absorbant en gonflement libre de 15 à 30 g de solution saline (0,9% de NaCl) par gramme de couche et une rétention de solution saline (0,9% de NaCl) absorbée de 5 à 20 g par gramme de couche.

7. Pansement pour plaie selon l'une quelconque de revendications 1 à 6, ayant une épaisseur de 2 à 4 mm.

8. Pansement pour plaie selon l'une quelconque des revendications 1 à 7, dans lequel soit le tissu d'alginate soit la couche comprenant le matériau superabsorbant contient aussi un agent anti-bactérien pour prévenir ou traiter une infection ou pour limiter la formation d'odeurs.

9. Pansement pour plaie selon l'une quelconque des revendications 1 à 8, dans lequel le matériau superabsorbant est un polyacrylate.

10. Pansement pour plaie selon l'une quelconque des revendications 1 à 9, dans lequel le pansement est un tissu aiguilleté unitaire d'une toile de fibre d'alginate et d'un tissu contenant un matériau superabsorbant fibreux.
